# EUROPEAN PATENT SPECIFICATION

(11) **EP 1 442 705 B1**
(45) Date of publication and mention of the grant of the patent: **28.02.2007**
(21) Application number: 03007455.3
(22) Date of filing: 03.04.2003
(51) Int. Cl.: A61B 5/15

(54) **Blood collection set with venting mechanism**
Blutentnahmeset mit Entlüftungsmechanismus
Ensemble de collecte de sang avec mécanisme d'aération

(30) Priority: 16.01.2003 US 440752 P
(43) Date of publication of application: 04.08.2004
(73) Proprietor: Becton, Dickinson and Company, Franklin Lakes, New Jersey 07417-1880 (US)
(72) Inventor: Marsden, Stewart E., Montville, New Yersey 07045 (US)
(74) Representative: Müller-Boré & Partner Patentanwälte

(56) References cited:
- EP-A- 0 376 168
- EP-A- 0 848 441
- WO-A-98/56825
- WO-A-99/23947
- US-A- 6 024 727
- US-A1- 2001 044 847

## Description

### 1. Field of the Invention

The subject invention relates to a blood collection set with self-venting features.

### 2. Description of the Related Art

Phlebotomy procedures often are carried out using a blood collection set. A typical blood collection set includes an IV needle assembly with an IV cannula that has a proximal end, a sharply pointed distal end and a lumen extending between the ends. The needle assembly also includes a plastic IV hub with a proximal end, a distal end, and a passage extending between the ends. The proximal end of the IV cannula is mounted in the passage of the IV hub so that the lumen through the IV cannula communicates with the passage through the IV hub. The needle assembly may further include a shield for shielding the IV cannula after use and a packaging cover for safely covering the IV cannula prior to use. Packaging covers typically are rigid tubes with a proximal end that can be telescoped over the IV cannula and frictionally engaged with the distal end of the IV hub. Shields for blood collection sets have taken many forms. Some shields are telescoped over the IV hub and can be moved from a proximal position where the cannula is exposed to a distal position where the cannula is shielded. Other shields are hinged to the IV hub and can be rotated from an open position where the IV cannula is exposed to a closed position where the IV cannula is shielded. A needle assembly for a blood collection set also may include two flexible wings that project transversely from the IV hub or from the shield. The wings can be folded into face-to-face relationship with one another to effectively define a handle that facilitates manipulation of the needle assembly. The wings then can be rotated away from one another and held against the skin of the patient.

Blood collection sets also include a length of flexible plastic tubing. The tubing has a distal end that is connected to the proximal end of the IV hub. The tubing also has a proximal end that is connected to a plastic fitting. Thus, fluid communication is provided between the lumen of the IV cannula and the plastic fitting at the proximal end of the flexible tubing. The plastic fitting may be a female luer fitting that can be connected to a male luer fitting. The fitting then can be placed in communication with a reservoir or container for collecting a sample of blood.

Phlebotomy procedures often employ evacuated tubes, such as the VACUTAINER^{®} brand of evacuated tubes sold by Becton Dickinson and Company. Evacuated tubes often are used with a tube holder that has a proximal end, a distal end, and a tubular side wall extending between the ends. The proximal end of the holder is widely open and is configured for slidably receiving the evacuated tube. The distal end of the holder typically includes an end wall with a mounting aperture. The mounting aperture includes internal threads or other mounting structures.

The tube holder may be used with a non-patient needle assembly that has a non-patient hub with external surface configurations for mounting in the mounting aperture of the holder. The non-patient needle assembly further includes a non-patient cannula extending proximally from the hub and a multiple sample sleeve telescoped over the non-patient cannula and mounted to the proximal end of the hub. The hub of the non-patient needle assembly can be threaded or otherwise engaged in the mounting aperture of the tube holder so that the non-patient needle and the multiple sample sleeve project into the tube receiving chamber of the holder. In many situations the non-patient needle is pre-mounted in the tube holder.

The blood collection set may be used by mounting the fitting at the proximal end of the flexible plastic tubing to the distal end of the hub of the non-patient needle assembly. The packaging shield that covers the non-patient cannula then may be removed, and the hub of the non-patient needle assembly may be engaged with the tube holder. The medical practitioner then grips the IV needle assembly and removes the packaging cover from the IV cannula. The gripping of the IV needle assembly may include folding the flexible wings into face-to-face engagement and gripping the folded wings between a thumb and forefinger. The pointed distal end of the IV cannula then is urged into a targeted blood vessel. The wings then may be folded into engagement with the skin of the patient and may be taped in position. An evacuated tube then is urged into the open proximal end of the blood collection tube holder so that the non-patient needle pierces the stopper of the evacuated tube. As a result, the blood vessel of the patient is placed in communication with the interior of the evacuated tube, and the pressure differential between the blood vessel and the evacuated tube will generate a flow of blood through the IV cannula, through the passage of the IV hub, through the flexible tubing, through the non-patient hub and finally through the non-patient needle and into the evacuated tube.

It will be appreciated that a significant volume of air must be displaced before blood enters the evacuated tube. This air will be displaced by the flowing blood and will be urged into the evacuated tube. The flow of air into the evacuated tube increases the air pressure in the tube and offsets the pressure differential that generates the flow of blood from the patient to the evacuated tube. Thus, blood flow is slowed. Blood flow into the blood collection tube may stop when the pressure in the tube equals the fluid pressure of the blood. Additionally, the air urged into the blood collection tube can react with the blood or with certain additives in the tube to affect test results that might be performed on the sample in the tube.

Medical practitioners have several approaches for addressing problems relating to air in a blood collection set at the start of a phlebotomy procedure. For example, the first tube of collected blood may be considered a discard tube. Thus, the evacuated tube will remain in communication with the non-patient needle until blood begins to flow into the tube. The tube then will be removed and discarded and a second tube will be inserted into the holder for collecting a sample that can be used reliably. This approach adds to the cost and time of the procedure and wastes blood. Some medical practitioners try to vent air from the system before the first blood collection tube is placed in communication with the non-patient needle. This approach also wastes blood and can lead to contamination or accidental sticks depending upon the method of venting.

The typical needle hub is formed from an opaque plastic material, and plastic tubing often is formed from a translucent plastic material. Neither the opaque plastic material nor the translucent flexible tubing provide a clear indication of venous or arterial access. Blood flow into an evacuated tube does provide an indication of venous or arterial access. However, the initial movement of air into the evacuated tube delays the flow of blood into the evacuated tube. Thus, a medical practitioner may have a delayed indication of venous or arterial access and may incorrectly assume that the blood vessel was not accessed properly. In these situations, the medical practitioner may try to access the blood vessel again even though the initial access was successful. Accordingly, the patient may be subjected to unnecessary trauma during a repeated attempt to access the targeted blood vessel.

WO 99/23947 discloses among others in figure 16 a fluid collection device according to the preamble of claim 1.

EP 0 376 168 A2 discloses a blood-sampling device comprising a coupling device including first and second ports without a needle in flow communication with a needle. The first port is connectable to a blood storage device and the second port is connectable to a syringe for temporarily storing a first portion of blood withdrawn from a patient.

US 6,024,727 discloses among others in figure 23 a catheter insertion apparatus having a hub and hydrophobic plug for providing a visually determinable blood "flash" which is used to ascertain entry of a needle into a blood vessel.

### SUMMARY OF THE INVENTION

The invention is a self-venting blood collection set with a self-venting mechanism that permits escape of air while preventing an outflow of fluid, such as blood. Thus, air under venous pressure will be allowed to escape from the blood collection set until blood reaches the venting mechanism. The venting mechanism then will seal to allow blood to be collected into evacuated collection tubes or into other appropriate blood collection receptacles. The venting mechanism may be formed from a hydrophobic material, such as carbon methyl cellulose and preferably is at a location in the blood collection set close to the location that will be placed in communication with the evacuated collection tube or other such container.

The blood collection set includes an IV needle assembly, a length of flexible plastic tubing extending from the IV needle assembly and a non-patient needle assembly. The venting mechanism is disposed on the non-patient needle assembly to permit venting of a maximum amount of the air that is in the blood collection set prior to the initiation of a phlebotomy procedure.

The IV needle assembly comprises an IV hub having a proximal end, a distal end and a passage extending between the ends. The IV needle assembly further comprises an IV cannula having a proximal end mounted in the passage of the IV hub, a pointed distal end projecting distally from the IV hub and a lumen that communicates with the passage through the IV hub. The flexible tubing is connected to the proximal end of the IV hub. The IV needle assembly may further include a packaging cover that protectively encloses the IV needle cannula prior to use. The packaging cover is removed immediately prior to use to permit access to the IV cannula. The IV needle assembly may further include a protective shield that is moveable relative to the IV cannula from an open position where the IV cannula is exposed to a closed position where the IV cannula is substantially shielded. The shield protects against accidental sticks with the used IV cannula. A pair of flexible wings may be mounted to the IV hub or to the shield to facilitate manipulation of the IV needle assembly.

The non-patient needle assembly includes a non-patient hub having a proximal end and a distal end. The non-patient needle assembly further includes a non-patient cannula having a distal end securely mounted in the passage through the non-patient hub, a proximal end projecting proximally from the non-patient hub and a lumen that communicates with the passage through the non-patient hub. A multiple sample sleeve may be mounted over the non-patient cannula and secured to the proximal end of the non-patient hub. External portions of the non-patient hub near the proximal end thereof may be formed with an array of external threads or other mounting structure to enable the non-patient needle assembly to be mounted to a collection tube holder or other such medical device. The distal end of the non-patient hub may have a male luer taper that can be placed in communication with a corresponding female luer fitting.

The blood collection set may further include a fitting mounted to the proximal end of the flexible plastic tubing and configured for mating with the distal end of the non-patient hub. For example, the fitting may be a female luer fitting that can be engaged with the male luer taper at the distal end of the non-patient hub. The venting mechanism may extend through the non-patient hub at a location near the distal end of the non-patient needle. The venting mechanism provides communication between the passage through the non-patient hub and the surrounding environment. Alternatively, the venting mechanism may be formed in the fitting mounted to the proximal end of the flexible tubing.

The venting mechanism may comprise a transverse aperture extending through the non-patient plug or through the fitting at the proximal end of the flexible tubing. Additionally, the venting mechanism may comprise a hydrophobic material, such as the above-referenced carbon methyl cellulose mounted in the venting aperture. The hydrophobic material permits air to pass through the hydrophobic material in response to the pressure of blood entering the blood collection set. The air will be urged through the hydrophobic material or other such venting mechanism until the blood reaches the venting mechanism. The blood will not flow through the hydrophobic material and will be at or near to the non-patient needle so that only a minimal amount of air will be collected with the first sample of blood.

The blood collection set of the invention can be used substantially in the conventional manner. In particular, the IV packaging cover is removed from the IV cannula and the medical practitioner accesses a targeted blood vessel with the pointed distal end of the IV cannula. Venous pressure will cause blood to flow through the IV needle assembly and into the flexible plastic tubing. The venous or arterial pressure exceeds the ambient air pressure existing in the flexible plastic tubing and other parts of the blood collection set. Hence, the flowing blood will urge air in the blood collection set out through the venting mechanism on or near the non-patient needle assembly. The hydrophobic material of the venting mechanism and the multiple sample sleeve over the non-patient needle will prevent blood from flowing beyond the blood collection set.

The medical practitioner then may remove the packaging cover from the non-patient needle assembly and may thread or otherwise connect the non-patient hub to the collection tube holder. An evacuated collection tube then can be slid into the open proximal end of the collection tube holder so that the lumen through the non-patient needle is placed in communication with the evacuated interior of the collection tube. The venous or arterial pressure will urge the blood into the collection tube with only a minimal amount of air. As a result, there will be no need for a discard tube or any other blood venting procedure that had been employed in the prior art. Other variations of the above-described blood collection procedure can be employed. For example, the non-patient needle assembly can be connected to the needle holder prior to accessing the blood vessel with the IV needle assembly.

### BRIEF DESCRIPTION OF THE DRAWINGS

FIG. 1 is a perspective view of a blood collection set and collection tube holder in accordance with the subject invention.

FIG. 2 is a top plan view of the blood collection set and collection tube holder shown in FIG. 1.

FIG. 3 is a perspective view of the non-patient needle assembly of the blood collection set.

FIG. 4 is a side elevational view of the non-patient needle assembly, partly in section.

FIG. 5 is a top plan view similar to FIG. 2, but showing an alternate embodiment of the invention.

### DETAILED DESCRIPTION

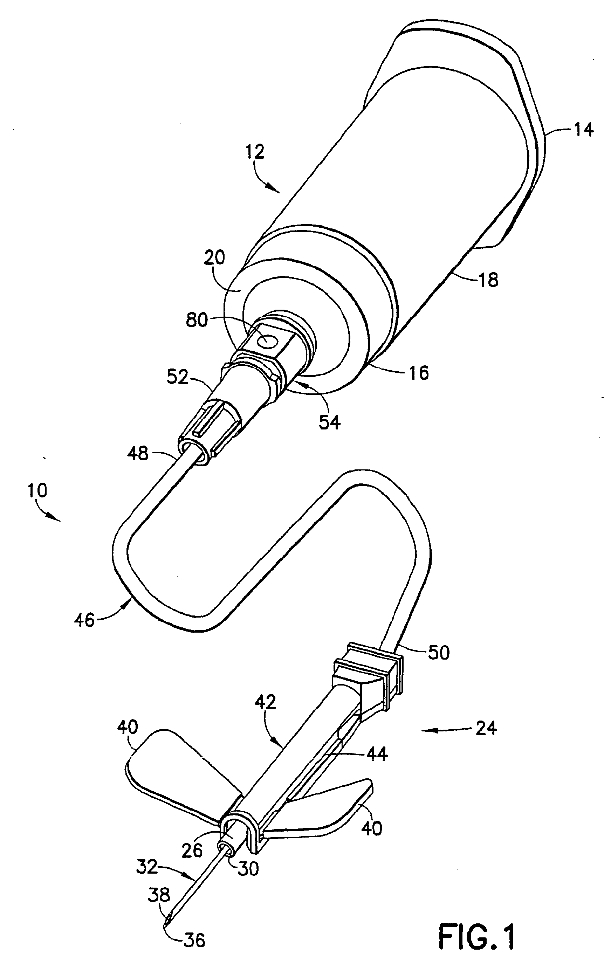

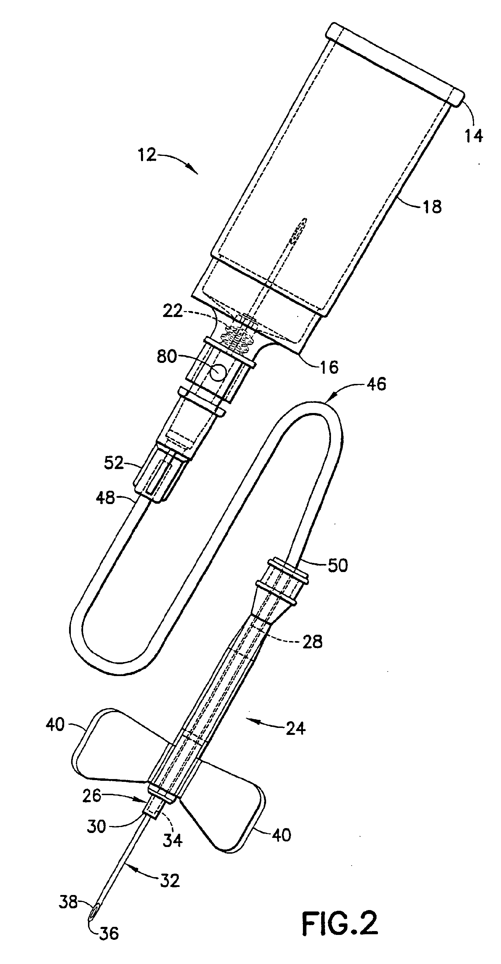

A blood collection set in accordance with the subject invention is identified generally by the numeral **10** in FIGS. 1 and 2. Blood collection set **10** is employed in this embodiment with a collection tube holder **12**. Holder **12** has a proximal end **14**, a distal end **16** and a tubular sidewall **18** extending between the ends. Proximal end **14** of holder **12** is widely open and defines an entry to a tube receptacle within sidewall **18**. Thus, an evacuated collection tube can be slid in a proximal-to-distal direction through open proximal end **14** of holder **12** toward distal end **16**. Distal end **16** of holder **12** is characterized by an end wall **20**. End wall **20** is formed with an internally threaded mounting aperture **22**, as shown in FIG. 2.

Blood collection set **10** includes an IV needle assembly **24** that comprises an IV hub **26**. IV hub **26** includes a proximal end **28**, a distal end **30** and a passage (not shown) extending between the ends. IV needle assembly **24** further includes an IV cannula **32** with a proximal end **34**, a pointed distal end 36 and a lumen **38** extending between the ends. Proximal end **34** of IV cannula **32** is mounted securely in the passage of IV hub **26**. Thus, lumen **38** through IV cannula **32** communicates with the passage through IV hub **26**. Flexible wings **40** are mounted to IV hub **26** at a location near distal end **30**. Wings **40** can be folded into face-to-face relationship with one another for convenient gripping between a thumb and forefinger to enable manipulation of IV needle assembly **24**. Wings **40**, however, also can be rotated into a substantially coplanar disposition for taping to the skin of a patient.

IV needle assembly **24** further includes a tubular shield **42** that is telescoped over IV hub **26**. Shield **42** is formed with transverse slots **44** that slidably receive wings **40**. Thus, shield **42** can be slid from a proximal position, as shown in FIGS. 1 and 2 to a distal position. IV cannula **32** is exposed for use when shield **42** is in the proximal position shown in FIGS. 1 and 2. However, IV cannula **32** is substantially surrounded by shield **42** when shield **42** is moved to the distal position. Additionally, slots **44** in shield **42** are configured to lockingly engage wings **40** when shield **42** is in the distal position to prevent or complicate a re-exposure of IV cannula **32**. The shield illustrated in FIGS. 1 and 2 is one of many optional shield designs that can be incorporated into blood collection set **10**. Other designs may provide wings mounted directly to the shield. Still other designs may provide a hinged shield mounted to IV hub **26**. In still other designs, a shield may be entirely separate from IV needle assembly **24** or a shield may not be provided at all.

Blood collection set **10** further includes a length of flexible plastic tubing **46**. Tubing **46** includes opposite proximal and distal ends **48** and **50** and a passage extending between the ends. Distal end **50** of tubing **46** is securely mounted to proximal end **28** of IV hub **26** so that the passage through IV hub **26** communicates with the passage through tubing **46**. A female luer fitting **52** is securely mounted to proximal end **48** of tubing **46**.

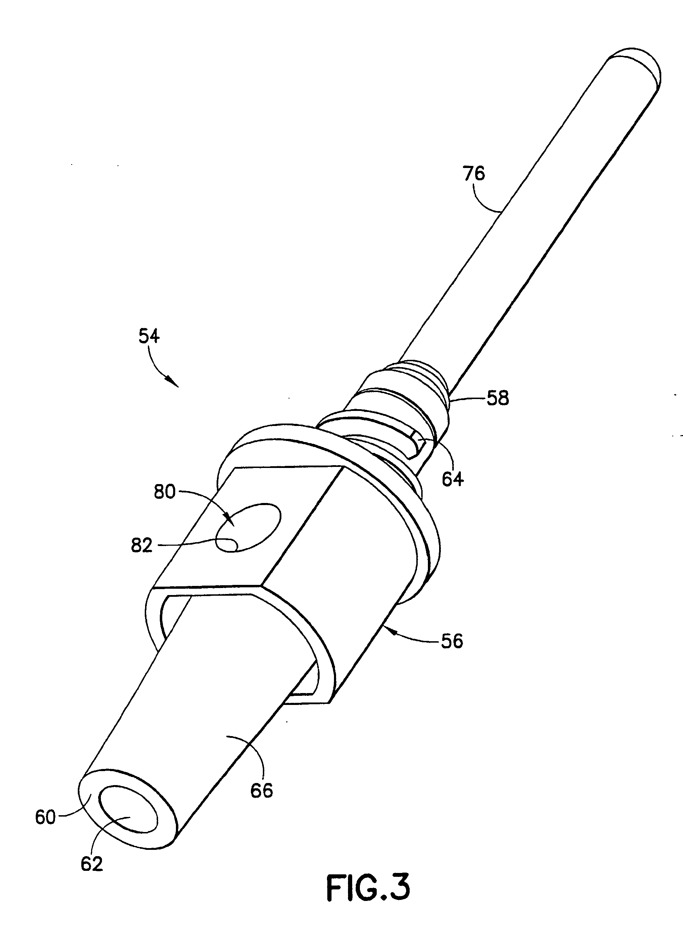

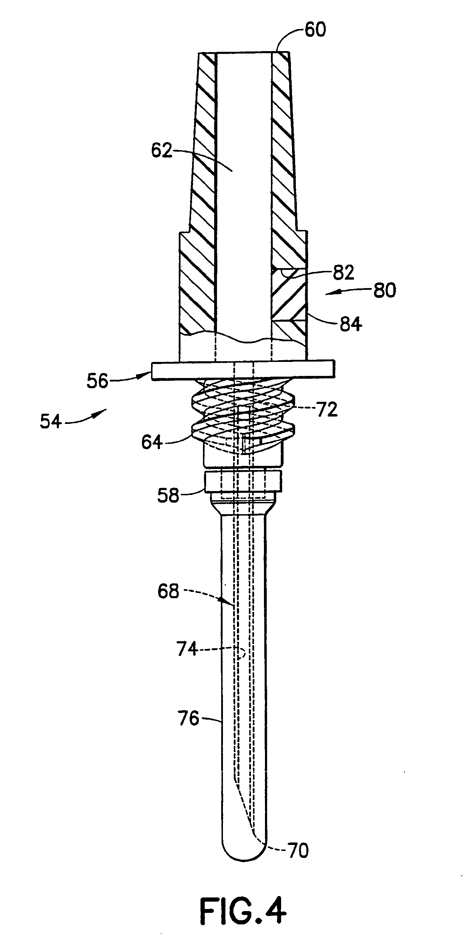

Blood collection set **10** further includes a non-patient needle assembly **54**, as shown most clearly in FIGS. 3 and 4. Non-patient needle assembly **54** includes a non-patient hub **56** with a proximal end **58**, a distal end **60** and an passage **62** extending between the ends. Exterior surface regions of non-patient hub **56** substantially adjacent proximal end **58** define an array of external threads **64** configured for threaded engagement with the internal threads formed in mounting aperture **22** of collection tube holder **12**. External surface regions of non-patient hub **56** adjacent distal end define a male luer taper **66** configured for mating with female luer fitting **52**. Non-patient needle assembly **54** further includes a non-patient cannula **68** having a pointed proximal end **70**, a distal end **72** and a lumen **74** extending between the ends. Distal end **72** of non-patient cannula **68** is mounted securely in passage **62** through non-patient hub **56** and aligns substantially with external threads **64** on non-patient hub **56**. Non-patient needle assembly **54** further includes a multiple sample sleeve **76** mounted over non-patient cannula **68** and securely engaged with proximal end **58** of non-patient hub **56.** Multiple sample sleeve **76** effectively functions as a valve that prevents a flow of fluid from non-patient cannula **68**. However, multiple sample sleeve **76** can be pierced by pointed proximal end **70** of non-patient cannula **68** in response to forces generated by a stopper on an evacuated collection tube.

Non-patient needle assembly **54** further includes a venting mechanism **80**. Venting mechanism **80** includes a transverse aperture **82** extending through non-patient hub **56** at a location between external threads **64** and luer taper **66**. Transverse aperture **82** provides communication between passage **62** of non-patient hub **56** and the ambient surroundings. As shown most clearly in FIG. 4, transverse aperture **82** is very close to distal end **72** of non-patient cannula **68**. Venting mechanism **80** further includes a venting plug **84** securely mounted in transverse aperture **82**. Venting plug **84** is formed from a hydrophobic material, such as carbon methyl cellulose (CMC). Alternatively, venting plug **84** may be formed from high-density polyethylene (HDPE), polytetrafluoroethylene (PTFE), ultra-high molecular weight polyethylene (UHMWPE), Nylon 6, polypropylene (PP), polyvinylidine fluoride (PVDF) or polyethersulfone (PES). Venting plug **84** permits an outflow of air, but prevents an outflow of blood or other fluids.

Blood collection set **10** is employed by folding wings **40** into face-to-face engagement with one another and gripping wings **40** between a thumb and forefmger. Any packaging cover that may be mounted over IV cannula **32** then is removed and discarded. Pointed distal end **36** of IV cannula **32** then is urged into a targeted blood vessel. The healthcare practitioner then may release the grip on wings **40,** and if long term access to the blood vessel is required, wings **40** may be taped into face-to-face engagement with the skin of the patient. Blood collection set **10** includes a plurality of internal spaces that will initially be at ambient air pressure. These internal spaces include lumen **38** through IV cannula **32**, the passage through IV hub **26**, the passage through flexible tubing **46**, passage **62** through non-patient hub **56** and lumen **74** through non-patient cannula **68**. The venous or arterial access achieved with IV cannula **32** places these interior spaces of blood collection set **10** in communication with the pressure of the blood in the patient. Blood pressure exceeds the ambient air pressure. Accordingly, the pressure of air in the above-referenced internal spaces will increase, and blood will begin to flow into these internal spaces. Prior art systems may reach equilibrium as the air pressure within the blood collection set increases in response to a reduction of volume caused by the inflow of blood. Hence, a portion of the internal spaces in the prior art system may remain filled with air at a pressure substantially equal to the venous or arterial pressure. Stated differently, a prior art system will include its original volume of air in the space between the proximal end of the non-patient needle and the blood that enters the blood collection set. This high-pressure air will escape into the first evacuated collection tube that is placed in communication with the non-patient needle. Hence, the first collection tube employed with prior art systems normally is a discard tube. With the subject invention, however, the communication of blood at venous or arterial pressure with the interior spaces of blood collection set **10** will urge air through venting mechanism **80**. Hence, the volume of air in the interior spaces of blood collection set **10** will decrease substantially without a substantial increase in air pressure. Blood will continue to flow into the interior spaces of blood collection set **10**, and particularly through the passage of flexible plastic tubing **46** and passage **62** of non-patient hub **56**. The outflow of air through venting mechanism **80** will terminate when blood reaches hydrophobic plug **84** of venting mechanism **80**. At this point, only a small volume of air will remain in portions of passage **62** between venting mechanism **80** and non-patient cannula **68**, as well as the small volumes of air in lumen **74** through non-patient cannula **68** and the volume of air between non-patient cannula **68** and the interior of multiple sample sleeve **76**. This volume of air existing proximally of venting mechanism **80** will be very small as compared to the volume of air that had been in flexible plastic tubing **46**, but was permitted to escape through venting plug **84**.

Use of blood collection set **10** proceeds substantially in a conventional manner by sliding an evacuated collection tube (not shown) into open proximal end **14** of collection tube holder **12**. The rubber stopper in the end of the collection tube will displace multiple sample sleeve **76** sufficiently for pointed distal end **70** of non-patient cannula **68** to pierce both multiple sample sleeve **76** and the stopper of the collection tube. A pressure differential then will exist again, and venous or arterial pressure will cause the small volume of air trapped proximally of the blood in non-patient hub **56** into the evacuated tube. Blood then will flow under venous or arterial pressure into the evacuated tube. The small volume of air that initially enters the evacuated tube generally will not require the initial tube to be discarded, and hence blood collection set **10** achieves lower cost and higher efficiency.

After the last sample of blood has been collected, IV cannula **32** is withdrawn from the patient and shield **42** is moved distally to shield IV cannula **32**. Blood collection set **10** then may be discarded in an appropriate sharps receptacle.

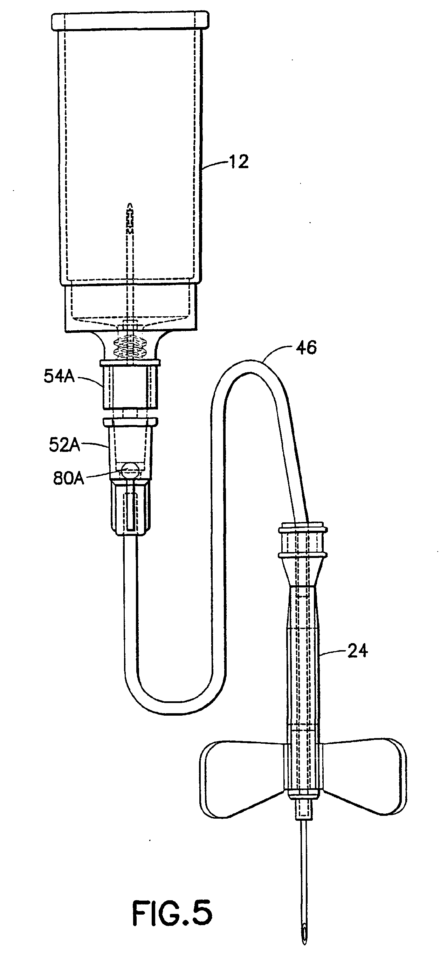

FIGS. 1-4 show an embodiment where venting mechanism **80** is formed in non-patient hub **56** of non-patient needle assembly **54**. The embodiment of FIGS. 1-4 provides venting plug **80** virtually as close as possible to the proximal end of blood collection set **10**. Similar effects can be achieved by providing venting mechanism **84A** in female luer fitting **52A** and a non-patient needle assembly **54A** with no vent, as shown in FIG. 5. The FIG. 5 embodiment is slightly less efficient than the embodiment of FIGS. 1-4 in that venting mechanism **84A** is spaced further from the proximal end of the blood collection set. However the FIG. 5 embodiment enables the use of prior art tube holders and prior art non-patient needle with much better performance than would be achieved with prior art blood collection sets.

## Claims

1. A blood collection set (10) comprising
an IV needle assembly (24) having an IV hub (26) and an IV cannula (32) mounted to said IV hub (26), said IV cannula (32) having a lumen (38) extending therethrough,
a length of flexible tubing (46) having opposite first (50) and second ends (48) and a passage extending between said ends (50, 48) of said flexible tubing (46), said first end (50) of said flexible tubing (46) being mounted to said IV hub (26) for providing communication between said lumen (38) of said IV cannula (32) and said passage through said flexible tubing (46),
a non-patient needle assembly (54) having a non-patient hub (56) being in fluid communication with of said second end (48) of said flexible tubing (46) and a non-patient cannula (68) having a pointed proximal end (70), a distal end (72) and a lumen (74) extending between said ends of said non-patient cannula (68), wherein said distal end (72) of said non-patient cannula (68) is securely mounted to said non-patient hub (56),
said non-patient hub (56) having a proximal end (58), a distal end (60) and a single passage (62) extending therebetween, said single passage (62) being in fluid communication with said lumen (74) through said non-patient cannula (68) and with said second end of said flexible tubing (46),
**characterized by**
a venting mechanism (80) extending through said non-patient hub (56) for providing communication between said single passage (62) through said non-patient hub (56) and ambient surroundings, said venting mechanism (80) permitting an outflow of air from said blood collection set (10) while preventing an outflow of fluid therefrom.

2. The blood-collection set (10) of Claim 1, wherein said venting mechanism (80) includes a hydrophobic material.

3. The blood-collection set (10) of Claim 2, wherein said hydrophobic material is carboxymethylcellulose.

4. The blood-collection set (10) of any of Claims 1 to 3, wherein the venting mechanism (80) includes a material selected from the group consisting of carboxymethylcellulose (CMC), high-density polyethylene (HDPE), polytetrafluoroethylene (PTFE), ultra-high molecular weight polyethylene (UHMWPE), Nylon 6, polypropylene (PP), polyvinylidine fluoride (PVDF) and polyethersulfone (PES).

5. The blood collection set (10) of any of Claims 1-4, further comprising a luer fitting (52) securely mounted to said second end (48) of said flexible tubing (46), wherein said non-patient hub (56) is secured to said luer fitting (52).

6. The blood collection set (10) of any of Claims 1 to 5, wherein said non-patient hub (56) includes a male luer taper (66) formed thereon.

7. The blood collection set (10) of Claim 6, wherein said luer fitting (52) is a female luer fitting (52) being mated with said male luer taper (66) of said non-patient hub (56).

8. The blood collection set (10) of any of Claims 1-7, further comprising a shield for shielding said IV cannula (32).

9. The blood collection set (10) of any of Claims 1-8, wherein said venting mechanism (80) extends through said non-patient hub (56) transversely of said passage (62).

10. The blood-collection set (10) of Claims 1 to 9, further comprising a multiple sample sleeve (76) enclosing said non-patient cannula proximal end (70) and securely mounted to said non-patient hub (56).

## Patentansprüche

1. Blutentnahmeset (10) umfassend
eine IV Nadelanordnung (24), die eine IV Nabe (26) und eine IV Kanüle (32) aufweist, die an der IV Nabe (26) festgelegt ist, wobei die IV Kanüle (32) ein Lumen bzw. Loch (38) aufweist, das sich **dadurch** erstreckt,
eine Länge eines flexiblen Rohrs bzw. einer Rohrleitung (46), das bzw. die gegenüberliegende erste (50) und zweite Enden (48) und einen Durchtritt aufweist, der sich zwischen den Enden (50, 48) der flexiblen Rohrleitung (46) erstreckt, wobei das erste Ende (50) der flexiblen Rohrleitung (46) an der IV Nabe (26) zum Bereitstellen einer Kommunikation bzw. Verbindung zwischen dem Lumen (38) der IV Kanüle (32) und dem Durchtritt durch die flexible Rohrleitung (46) montiert bzw. angeordnet ist,
eine Nicht-Patienten-Nadelanordnung (54), die eine Nicht-Patienten-Nabe (56), die in Fluidverbindung mit dem zweiten Ende (48) der flexiblen Rohrleitung (46) ist, und eine Nicht-Patienten-Kanüle (68) aufweist, die ein zugespitztes proximales Ende (70), ein distales Ende (72) und ein Lumen bzw. Loch (74) aufweist, das sich zwischen den Enden der Nicht-Patienten-Kanüle (68) erstreckt, wobei das distale Ende (72) der Nicht-Patienten-Kanüle (68) sicher an der Nicht-Patienten-Nabe (56) festgelegt ist,
wobei die Nicht-Patienten-Nabe (56) ein proximales Ende (58), ein distales Ende (60) und einen einzigen Durchtritt (62) aufweist, der sich dazwischen erstreckt, wobei der einzige Durchtritt (62) in Fluidwechselwirkung bzw. -verbindung mit dem Lumen (74) durch die Nicht-Patienten-Kanüle (68) und mit dem zweiten Ende des flexiblen Rohrs (46) ist,
**gekennzeichnet durch**
einen Belüftungsmechanismus (80), der sich **durch** die Nicht-Patienten-Nabe (56) zum Bereitstellen einer Verbindung zwischen dem einzigen Durchtritt (62) **durch** die Nicht-Patienten-Nabe (56) und umliegende Umgebungen erstreckt, wobei der Belüftungsmechanismus (80) einen Ausfluß von Luft aus dem Blutsammel- bzw. -entnahmeset bzw. -satz (10) erlaubt, während ein Ausfluß von Fluid davon verhindert ist.

2. Blutentnahmeset (10) nach Anspruch 1, wobei der Belüftungsmechanismus (80) ein hydrophobes Material beinhaltet.

3. Blutentnahmeset (10) nach Anspruch 2, wobei das hydrophobe Material Carboxymethylzellulose ist.

4. Blutentnahmeset (10) nach einem der Ansprüche 1 bis 3, wobei der Belüftungsmechanismus (80) ein Material enthält, gewählt aus der Gruppe bestehend aus Carboxymethylcellulose (CMC), hochdichtem Polyethylen (HDPE), Polytetrafluorethylen (PTFE), Ultrahochmolekulargewicht-Polyethylen (UHMWPE), Nylon 6, Polypropylen (PP), Polyvinylidinfluorid (PVDF) und Polyethersulfon (PES).

5. Blutentnahmetest (10) nach einem der Ansprüche 1-4, weiterhin umfassend einen Luer-Fitting bzw. ein Luer-Paßstück (52), der bzw. das sicher an dem zweiten Ende (48) der flexiblen Rohrleitung (46) festgelegt ist, wobei die Nicht-Patienten-Nabe (56) an dem Luer-Fitting (52) gesichert ist.

6. Blutentnahmetest (10) nach einem der Ansprüche 1 bis 5, wobei die Nicht-Patienten-Nabe (56) eine aufzunehmende Luer-Verjüngung (66) beinhaltet, die darauf ausgebildet ist.

7. Blutentnahmetest (10) nach Anspruch 6, wobei das Luer-Paßstück (52) ein aufnehmendes Luer-Paßstück (52) ist, das mit der aufzunehmenden Luer-Verjüngung (66) der Nicht-Patienten-Nabe (56) zusammenpaßt.

8. Blutentnahmetest (10) nach einem der Ansprüche 1-7, weiterhin umfassend einen Schirm bzw. eine Abschirmung zum Abschirmen der IV Kanüle (32).

9. Blutentnahmetest (10) nach einem der Ansprüche 1-8, wobei sich der Belüftungsmechanismus (80) durch die Nicht-Patienten-Nabe (56) quer von dem Durchtritt (62) erstreckt.

10. Blutentnahmetest (10) nach Ansprüchen 1 bis 9, weiterhin umfassend eine Mehrfachprobenhülse (76), die das proximale Ende der Nicht-Patienten-Kanüle (70) umschließt und sicher an der Nicht-Patienten-Nabe (56) festgelegt ist.

## Revendications

1. Ensemble de collecte de sang (10) comprenant :
un dispositif d'aiguille intraveineuse IV (24) ayant un moyeu IV (26) et une canule IV (32) fixée au dit moyeu (26), la dite canule IV (32) ayant une lumière (38) qui s'étend le long de la canule,
une longueur de tube souple (46) ayant une première (50) et une deuxième (48) extrémités opposées et un passage s'étendant entre les dites extrémités (50, 48) du dit tube souple (46), la dite première extrémité (50) du dit tube souple (46) étant fixée au dit moyeu IV (26) pour créer une communication entre la dite lumière (38) de la dite canule IV (32) et le dit passage à l'intérieur du dit tube souple (46),
un dispositif d'aiguille non de patient (54) ayant un moyeu non de patient (56) en communication de fluide avec la dite deuxième extrémité (48) du dit tube souple (46) et une canule non de patient (68) ayant une extrémité proximale pointue (70), une extrémité distale (72) et une lumière (74) s'étendant entre les dites extrémités de la dite canule non de patient (68), dans lequel la dite extrémité distale (72) de la dite canule non de patient (68) est fixée de façon sûre au dit moyeu non de patient (56),
le dit moyeu non de patient (56) ayant une extrémité proximale (58), une extrémité distale (60) et un passage unique (62) s'étendant entre ces extrémités, le dit passage unique (62) étant en communication de fluide avec la dite lumière (74) de la dite canule non de patient (68) et avec la dite deuxième extrémité du dit tube souple (46),
**caractérisé par**
un mécanisme d'aération (80) s'étendant à travers le dit moyeu non de patient (56) pour créer une communication entre le dit passage unique (62) du dit moyeu non de patient (56) et l'environnement ambiant, le dit mécanisme d'aération (80) permettant une sortie d'air du dit ensemble de collecte de sang (10) tout en empêchant une sortie de fluide de cet ensemble.

2. Ensemble de collecte de sang (10) selon la revendication 1, dans lequel le dit mécanisme d'aération (80) comprend une matière hydrophobe.

3. Ensemble de collecte de sang (10) selon la revendication 2, dans lequel la dite matière hydrophobe est la carboxyméthyl cellulose.

4. Ensemble de collecte de sang (10) selon une quelconque des revendications 1 à 3, dans lequel le mécanisme d'aération (80) comprend une matière choisie dans le groupe composé de carboxyméthyl cellulose (CMC), polyéthylène haute densité (HDPE), polytétrafluoro éthylène (PTFE), polyéthylène d'ultra-haut poids moléculaire (UHMWPE), nylon 6, polypropylène (PP), fluorure de polyvinylidène (PVDF) et polyéther sulfone (PES).

5. Ensemble de collecte de sang (10) selon une quelconque des revendications 1 à 4, comprenant en outre un raccord luer (52) fixé de façon sûre à la dite deuxième extrémité (48) du dit tube souple (46), dans lequel le dit moyeu non de patient (56) est fixé au dit raccord luer (52).

6. Ensemble de collecte de sang (10) selon une quelconque des revendications 1 à 5, dans lequel le dit moyeu non de patient (56) comprend un cône luer mâle (66) formé sur le moyeu.

7. Ensemble de collecte de sang (10) selon la revendication 6, dans lequel le dit raccord luer (52) est un raccord luer femelle (52) accouplé au dit cône luer mâle (66) du dit moyeu non de patient (56).

8. Ensemble de collecte de sang (10) selon une quelconque des revendications 1 à 7, comprenant en outre un fourreau pour protéger la dite canule IB (32).

9. Ensemble de collecte de sang (10) selon une quelconque des revendications 1 à 8, dans lequel le dit mécanisme d'aération (80) s'étend à travers le dit moyeu non de patient (56) transversalement au dit passage (62).

10. Ensemble de collecte de sang (10) selon une quelconque des revendications 1 à 9, comprenant en outre un manchon d'échantillon multiple (76) encerclant l'extrémité proximale de la dite canule non de patient (70) et monté de façon fixe sur le dit moyeu non de patient (56).
